(19)

**Europäisches Patentamt**
**European Patent Office**
**Office européen des brevets**

(11) **EP 4 702 931 A1**

(12) **EUROPEAN PATENT APPLICATION**

(43) Date of publication:
04.03.2026 Bulletin 2026/10

(21) Application number: 24198082.0

(22) Date of filing: 03.09.2024

(51) International Patent Classification (IPC):
*A61B 6/04* (2006.01)   *A61B 6/00* (2024.01)
*A61B 6/58* (2024.01)   *G06T 7/73* (2017.01)

(52) Cooperative Patent Classification (CPC):
**A61B 6/488; A61B 6/0492; A61B 6/582;**
**A61B 6/584; G06T 7/73;** G06T 2207/10116;
G06T 2207/20081; G06T 2207/20084;
G06T 2207/30012

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: Siemens Healthineers AG
91301 Forchheim (DE)

(72) Inventors:
• **Biniazan, Ramyar**
  **90402 Nürnberg (DE)**
• **Fieselmann, Andreas**
  **91052 Erlangen (DE)**
• **Fok, Wai Yan Ryana**
  **85748 Garching b. München (DE)**
• **Hümmer, Christian**
  **96215 Lichtenfels (DE)**

(74) Representative: **Siemens Healthineers**
**Patent Attorneys**
**Postfach 22 16 34**
**80506 München (DE)**

(54) **METHOD AND DEVICE FOR OPTIMIZING THE POSITIONING OF A PATIENT FOR X-RAY PROJECTION IMAGING**

(57)   The invention pertains to a method for optimizing the positioning of a patient (P) for X-ray projection imaging, comprising the steps:
- providing a base-image (BI) of a body part of the patient (P) positioned for acquiring the projection image,
- identifying from a predefined set of landmarks a number of center-landmarks (LC) and a plurality of edge-landmarks (LE) in the base-image (BI), wherein each center-landmark (LC) lies between at least two edge-landmarks (LE) and wherein the equivalents of the landmarks in the patient (P) lie on a number of triangles inclined to the projection plane of the base-image (BI),
- determining distances (D1, D2) of at least one center-landmark (LC) to the at least two edge-landmarks (LE) in the base-image (BI),
- calculating a ratio-value ($\rho$) of the calculated distances (D1, D2),
- determining a patient-rotation angle ($\alpha$) from the ratio-value ($\rho$) and a given mapping-function (F) that maps the ratio-value ($\rho$) to a patient-rotation angle ($\alpha$),
- generating output-data (O) based on the determined patient-rotation angle ($\alpha$),
- outputting the generated output-data (O).

Furthermore, the invention describes a device and a medical X-ray-system.

FIG 2

**Description**

The invention describes a method and a device for optimizing the positioning of a patient for X-ray projection imaging and a medical X-ray-system.

Automatic patient positioning plays a vital role during X-ray examination. In fact, having good patient position ensures diagnostic accuracy and supports image analysis. In case of a poorly positioned patient, an information is missing regarding the detailed explanation of how to achieve a correct position of the patient.

There are known systems for a patient positioning check consisting of two main components. The first component is describing a rule-based patient positioning check in a generic way. That component consists of the steps segmenting structures from an image and rule-based assessing of the segmented structures.

The second component is an end-to-end approach to quantify the internal patient rotation.

The first component is transparent (the segmented structured can be shown to the user) but not quantitative (the rotation angle is not quantified), the second component is quantitative (it is trained to regress the rotation angle) but not transparent (as it is an end-to-end approach). This could be a problem, since there is no transparent quantification.

It is the object of the present invention to improve the known systems and methods and provide a method and a device for optimizing the positioning of a patient for X-ray projection imaging and a medical X-ray-system, for overcoming the above described problems. Especially, it is an Object of the present invention to provide a feedback loop to train a technologist for an X-ray examination setup so the examination workflow can be improved and biomedical or clinical workload for determining if and how re-take of X-ray examination is needed can be reduced.

This object is achieved by a method according to claim 1, a device according to claim 10 and a medical X-ray-system according to claim 12.

The invention has the aim to combine both components in order to provide a system that offers quantitative (its correction information) as well as transparent properties.

A method according to the invention serves for optimizing the positioning of a patient for X-ray projection imaging. It comprises the following steps:

- providing a base-image of a body part of the patient positioned for acquiring the projection image,
- identifying from a predefined set of landmarks a number of center-landmarks and a plurality of edge-landmarks in the base-image, wherein each center-landmark lies between at least two edge-landmarks and wherein the equivalents of the landmarks in the patient lie on a number of triangles inclined to the projection plane of the base-image,
- determining distances of at least one center-landmark to the at least two edge-landmarks in the base-image,
- calculating a ratio-value of the calculated distances,
- determining a patient-rotation angle from the ratio-value and a given mapping-function that maps the ratio-value to a patient-rotation angle,
- generating output-data based on the determined patient-rotation angle,
- outputting the generated output-data.

The method serves for giving advice how to position a patient for acquiring an X-ray projection image, then the base-image could be a low intensity pilot image recorded before a main image. It could also be used for correcting an image or measurements taken from an image. In this case also the main image could be used as base-image. All measurements made in this image that would be affected by false positioning could be corrected with the output data, since this is based on the (correct) patient-rotation angle. In the case the base-image (the image acquired) passes the positioning check, it could be used as a main image that could be examined. If not, a further image could be acquired, until the patient is correctly positioned.

The patient-rotation angle is an angle in a predefined coordinate system ("reference system"). This reference system is preferably a cartesian coordinate system and should relate to the coordinate system of the imaging system. Particularly preferably, the X- and Y-axis lie in a horizontal plane and the Z-axis is arranged vertically. In this reference system, the patient-rotation angle reflects the arrangement of the coordinate system of the patient ("patient system") to the reference system. In a preferred patient system, the X-axis is the longitudinal axis, the Y-axis is the frontal axis and the Z-axis is the sagittal axis. For many examinations, one single angle (e.g. according to the Z-axis) could be enough as patient-rotation angle. However, in general, the rotation can occur around any of the 3 spatial axes. This means that the patient-rotation angle may have one (angle to one axis), two (two angles, each to a different axis) or three entries (angles to X-, Y and Z-axis). Regarding a special axis: if the patient is positioned such that an axis of the patient system is parallel to the respective axis of the reference system, the respective entry of the patient-rotation angle is 0°. In cases the patient system should lie parallel to the reference system, the patient-rotation angle is a measure for deviation of the patient. In a case where the correct position of the patient would include a given position-angle of an axis of the patient system to the reference system (e.g. patient lying on the side with the sagittal axis being 90° (position-angle) to the Z-axis) a difference between this position angle (here e.g. 89°) and the patient-rotation angle would be a measure for the deviation of the patient (in this example 1°).

First, a base-image of a body part of the patient positioned for acquiring the projection image has to be provided. This could include acquiring this base-image or retrieving the base-image from a database (e.g. via PACS). A base-image could be a (low intensity) pilot-image or the main image of an examination. The base-image shows the patient arranged in the position to be (or being) examined, in order to determine the patient-rotation angle during the imaging process.

In this base-image, at least three landmarks are identified. For example, for an examination of the chest, points on the clavicles, especially inner points and a point on the vertebral column could be the three landmarks. Since the region of interest is given, it is easy to provide a predefined set of landmarks, where the landmarks could be chosen from.

One of three landmarks is a center-landmark and the two others are edge-landmarks. In the case there are more than three landmarks, there could be further center and/or edge-landmarks. However, independent on how much landmarks there are identified, each center-landmark is lying between at least two edge-landmarks. This means that in reality, the center-landmark lies between the at least two edge-landmarks.

Looking at three landmarks, one center-landmark and two edge-landmarks, they lie in a triangle in a patient (in 3D-space). Concerning the 2D projection plane of the base-image, this triangle is inclined to this projection plane. This means that while acquiring the base-image, in the body of the patient the center-landmark lies over or under the line between the two edge-landmarks. Thus, when the Patient rotates, the center-landmark on the projection plane (in a series of base images) seems to move relative to the edge-landmarks. Thus, the position of the center-landmark relative to the edge-landmarks provides a measure for the patient-rotation angle.

For estimating a patient-rotation angle around one axis, two edge-landmarks would be enough, for all three axes, one center-landmark and three edge-landmarks would be sufficient. The three edge-landmarks should form a triangle on the projection plane (i.e. in the base-image). From the turning of the three edge-landmarks, a patient-rotation angle around the Z-axis could be derived and patient-rotation angles around the X-axis and Y-axis could be derived from the position of the center-landmark relative to the three edge-landmarks. The center-landmark and the three edge-landmarks form three triangles in total forming a tetraeder.

One should note that in theory, when the triangle is well known, the patient-rotation angle could directly be calculated from the position of a center-landmark to the edge-landmarks since the ratio of distances from the center-landmark to the edge-landmarks would be a direct measure for the patient-rotation angle. However, in reality, the triangle is not always known. Also symmetries could not be used in any case (e.g. for imaging the chest), since the projection of the body of the patient will deform with the patient-rotation angle.

Thus, after calculating the distances of the center-landmark to the at least two edge-landmarks in the base-image and determining a ratio-value of the calculated distances, this ratio-value is not directly taken as a measure of the patient-rotation angle, but the given mapping-function is used to determine the patient-rotation angle from the ratio-value. This mapping-function maps the ratio-value to the patient-rotation angle. It should be noted that the expression "ratio-value" indicates that the value is based on the ratio of distances. It could especially be calculated by a difference and/or by a division.

The mapping function could be created by a machine learning model trained with base-images of a patient and given patient-rotation angles as ground truth, wherein it is preferred that the base-images are synthetic images of a 3D-model of a patient lying in many different poses with given patient-rotation angles on a surface. Thus, a preferred approach for training is to generate synthetic X-ray images (training images) with respect to the patient-rotation angles, then by knowing the true patient-rotation angle in each training image, the ratio-value will be calculated and finally by having a series of these two parameters ratio-values and respective patient-rotation angles, an accurate mapping function could be found, e.g. by fitting a polynomial through the value-pairs.

With the determined patient-rotation angle it could be estimated whether the patient is well arranged or not. For example, when the chest of the patient should be examined and the patient lies exactly on the back with the patient-rotation angle of 0°, the arrangement is optimal. When the patient-rotation angle derives from 0°, then a re-arrangement could be done.

For that, the output-data is generated based on the determined patient-rotation angle. This output data could be a message for a user and could only comprise the patient-rotation angle. The output may also be used for automatic arrangement and comprise commands designed to control an automatic positioning mechanism.

This generated output-data is then outputted. It could be shown on a display or sent to said automatic positioning mechanism.

This method provides a transparent and easy approach with only one component that gives both transparent and quantitative results. Even when AI is used, the clear steps of the method and especially the use of the mapping function always gives an insight into the determination of the patient-rotation angle.

A device according to the invention serves for optimizing the positioning of a patient for X-ray projection imaging. It comprises the following components:

- a data-interface, designed for receiving a base-image of a body part of the patient positioned for acquiring the projection image,
- a landmark-unit, designed for identifying from a predefined set of landmarks a number of center-landmarks and a plurality of edge-landmarks in the base-image, wherein each center-landmark lies between at least two edge-landmarks and wherein the equivalents of the landmarks in the patient lie on a triangle inclined to the projection plane of the base-image,
- a distance-unit, designed for determining distances of a center-landmark to at least two edge-landmarks in the base-image,
- a ratio-unit, designed for calculating a ratio-value of the calculated distances,
- a correction-unit, designed for determining a patient-rotation angle from the ratio value and a given mapping-function that maps the ratio-value to a patient-rotation angle,
- an output-unit, designed for generating an output based on the determined patient-rotation angle,
- a data-interface, designed for outputting the generated output.

In praxis, considering a certain type of image view, e.g. frontal, the anatomical structures would be in corresponding positions which are defined by clinical guidelines. The guidelines are usually observed by biomedical technicians and clinicians during the examination.

In the example use case of a chest X-ray, there are 4 criteria that define a well-positioned chest X-ray. One of these criteria is the spinous processes of the thoracic vertebrae should form a vertical line that lies equidistant from the medial ends of the clavicles. This distance ratio (the ratio-value p) could be calculated from the shortest distance A of a point of the left clavicle medial end to the vertebral column and the shortest distance B of a point of the right clavicle medial end to the vertebral column as: $p = (B - A)/(|B| + |A|)$.

At least for symmetric positioning, it is preferred to choose the ratio-value such that $p = 0$ when $A = B$ and ranges from -1 to 1. It is further preferred that the ratio-value ($p$) is a dimensionless quantity without explicit physical meaning.

In order to calculate the patient-rotation angle $\alpha$ the mapping function is needed. In order to create this mapping function, it is preferred to compute synthetic X-ray images from a CT volume as training images for several different patient-rotation angles $\alpha_G$ wherein the $\alpha_G$ are later used as ground truth values. In these training images, the relevant anatomical structures (e.g. clavicle, vertebral column) are segmented automatically (segmentation in 3D and forward projection to 2D). Based on these structures, the ratio-value p is computed. That process is repeated for many different combinations of CT volumes and $\alpha_G$ (may be more than 1000). From all samples, a mapping function is fitted (e.g. using a polynomial of $3^{rd}$ order). Also, a confidence interval can be created.

This gives a function $p = f(\alpha)$ which can be inverted to give the mapping function $\alpha = f^1(\rho)$. From the mapped alpha value, a category (e. g. good / bad positioning) can be determined (e. g. by thresholding).

This method can be applied to all body parts which are susceptible to positioning issues. This could be chest, hip, knee, femur or head. In this case, synthetic data may be created based on the body part of interest and later the ratio-value p could be defined based on clear measurements in the image as ration of distances between given landmarks and finally a mapping function could be found between p and $\alpha$.

The device is preferably designed for performing the method according to the invention. The function of the components of the device is described above.

A medical X-ray-system according to the invention comprises a device according to the invention and/or is designed for performing the method according to the invention.

Some units or modules of the invention mentioned above can be completely or partially realized as software modules running on a processor of a computing system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the methods, at least those steps that could be executed by a computer, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

According to a preferred method, parts of the body of the patient are segmented in the base-image, especially organs and/or bones, preferably by using a machine-learning-model trained for segmentation. The technic of segmenting an image is well known in the art. It is very advantageous for the invention in order to find the landmarks.

According to a preferred method, the distance of the center-landmark to a first edge-landmark is A and the distance of the center-landmark to a second edge-landmark is B and the ratio-value R is calculated with the difference of A and B normed with the sum of A+B in order to lie in a range between -1 and 1 ($R = (A-B)/(A+B)$ or $R = (B-A)/(A+B)$). It should be noted that this calculation of the ratio-value is advantageous for symmetric cases. For asymmetric cases, other formulas could be used to calculate the ratio-value. Due to the use of the mapping function, the formula to calculate the ratio-value is not crucial, but it is crucial to use the same formula to calculate the ratio-value from the base-image that has been used to determine the ratio-values used for the mapping function.

According to a preferred method, the patient-rotation angle is the angle of aberration of the patient from a desired rotation-angle. This is just a matter of choosing an appropriate coordinate system for the patient-rotation angle. For example, in a symmetric case, the coordinate system should be chosen such that the patient-rotation angle is 0° when the patient is arranged correctly. Instead of choosing a coordinate system, the mapping function could also be designed in an appropriate manner. For example, when an angle of 10° would be the correct angle, the mapping function could be designed such that the 10° are subtracted from the patient-rotation angle such that again 0° indicates a correct positioning.

According to a preferred method, the patient-rotation angle is determined with a machine-learning-model designed to output a patient-rotation angle based on an input of a ratio-value that has been trained by inputting sets of ratio-values with given real patient-rotation angles as ground truth values. As already described above, it is preferred that for training, multiple synthetic X-ray images (especially CT-images) of at least one body-part of a patient are generated that are projected in various given rotation angles, wherein for each synthetic X-ray image landmarks are detected, a ratio-value is calculated from distances between the landmarks and a patient-rotation angle is given as ground truth value based on the given rotation angle of the projection on the respective synthetic X-ray image.

According to a preferred method, matching patient-rotation angles are calculated for a plurality of certain ratio-values and a matching function is fitted to the certain values. It is preferred that the matching function is a polynomial greater than second order, preferably greater than third order. Particularly, it is preferred that the inverse function of the matching function is calculated as mapping-function for determining a patient-rotation angle from a ratio-value.

According to a preferred method, the body-part of the patient is the chest, the hip, the knee, shoulder, foot, the femur or the head.

According to a preferred method, the patient-rotation angle is calculated with a given mapping-function providing values for a desired patient-rotation angle from a certain ratio-value. It is preferred that the mapping-function is monotonically increasing or decreasing and crosses the origin.

A preferred method comprises the steps:

- providing a base-image of a chest of the patient,
- identifying at least a landmark on each clavicle and one landmark on the vertebral column in the base-image,
- calculating a distance A of the landmark on the vertebral column to the landmark on the right clavicle and a distance B of the landmark on the vertebral column to the landmark on the left clavicle in the base-image,
- calculating a ratio-value R of the calculated distances, preferably with the formula

$$R = (A-B)/(A+B) \text{ or } R = (B-A)/(A+B),$$

- determining a patient-rotation angle from the ratio value, preferably by using a given mapping-function,
- generating an output based on the determined patient-rotation angle,
- outputting the generated output.

A preferred device comprises a function-unit designed for generating a mapping-function from multiple calculations of patient-rotation angles from inputted ratio-values. It is preferred that the device, especially the function unit, comprises a machine-learning-model, trained for calculating a patient-rotation angle from an inputted ratio-value.

The use of AI-based methods (AI: "artificial intelligence") is preferred for the method according to the invention, especially for the segmentation of images, finding of landmarks or creating of the mapping function. Artificial intelligence is based on the principle of machine-based learning and is usually carried out with an adaptive algorithm that has been trained accordingly. The expression "machine learning" is often used for machine-based learning, which also includes the principle of "deep learning".

The methods may also include elements of "cloud computing". In the technical field of "cloud computing", an IT infrastructure is provided over a data-network, storage space or processing power and/or application software. The communication between the user and the "cloud" is achieved by means of data interfaces and/or data transmission protocols. In the context of "cloud computing", in a preferred embodiment of the methods according to the invention, provision of data via a data channel (for example a data-network) to a "cloud" takes place. This "cloud" includes a (remote) computing system, e.g. a computer cluster that typically does not include the user's local machine. It is particularly preferred that the cloud service provides as well computing power as application software.

Other objects and features of the present invention will become apparent from the following detailed descriptions considered in conjunction with the accompanying drawings. It is to be understood, however, that the drawings are designed solely for the purposes of illustration and not as a definition of the limits of the invention.

Figure 1 shows an X-ray-system with a device according to the invention,

Figure 2 shows a block diagram of the method according to the invention,

Figure 3 shows a segmentation procedure and an identification of landmarks,

Figure 4 shows the segmentation with a trained segmentation-model,

Figure 5 shows the training of a machine-learning-model producing a mapping function.

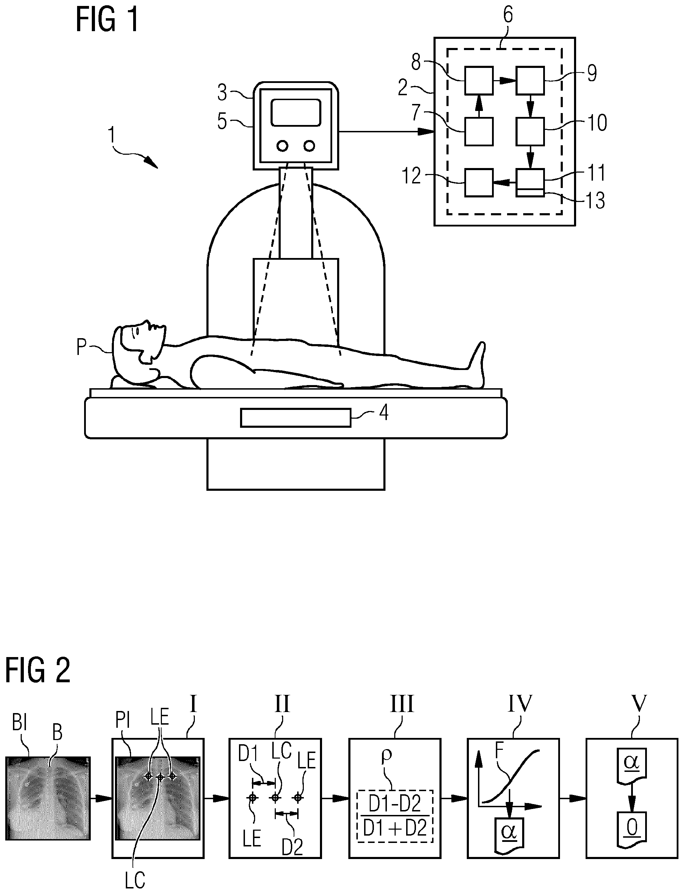

Figure 1 shows a schematic of an X-ray system 1 with a control-device 2. This is equipped with a device 6 designed to carry out the method according to the invention. The X-ray system 1 has, in the usual way, an X-ray source 3 and, while recording a projection image, irradiates a patient P with a beam collimated by the collimator 5, so that the radiation falls on a detector-unit 4 opposite the X-ray source 3.

In the control device 2, only the components that are essential for explaining the invention are shown. In principle, X-ray systems 1 and associated control-devices 2 are known to those skilled in the art and therefore do not need to be explained in detail.

The control device 2 comprises a device 6 for optimizing the positioning of a patient for X-ray projection imaging. It comprises a data-interface 7, a landmark-unit 8, a distance-unit 9, a ratio-unit 10, a correction-unit 11 (with a function-unit 13) and an output-unit 12.

The data-interface 7 is designed for receiving a base-image BI of a body part of the patient P positioned for acquiring the projection image, and for outputting the generated output at the end.

The landmark-unit 8 is designed for identifying from a predefined set of landmarks a number of center-landmarks LC and a plurality of edge-landmarks LE in the base-image BI, wherein each center-landmark LC lies between at least two edge-landmarks LE and wherein the equivalents of the landmarks in the patient P lie on a triangle inclined to the projection plane of the base-image BI.

The distance-unit 9 is designed for calculating the distances D2 D1 of the center-landmark LC to the at least two edge-landmarks LE in the base-image BI.

The ratio-unit 10 is designed for calculating a ratio-value p of the calculated distances D2 D1.

The correction-unit 11 is designed for determining a patient-rotation angle $\alpha$ from the ratio value and a given mapping-function F that maps the ratio-value p to a patient-rotation angle $\alpha$.

The output-unit 12 is designed for generating an output based on the determined patient-rotation angle $\alpha$.

Figure 2 shows a block diagram of the method for optimizing the positioning of a patient for X-ray projection imaging.

First (left), a base-image BI of a body part of the patient P positioned for acquiring the projection image is provided, e.g. recorded or downloaded from a database.

In Step I, the base-image BI is segmented and landmarks are identified from a predefined set of landmarks. Here one center-landmark LC on the vertebral column and two edge-landmarks LE on the clavicles are identified, in the base-image BI.

In Step II, the distances D2 D1 of the center-landmark LC to the two edge-landmarks LE are determined in the base-image BI.

In Step III, a ratio-value p of the calculated distances D2 D1 is calculated.

In Step IV, a patient-rotation angle $\alpha$ is determined from the ratio-value p and a given mapping-function F that maps the ratio-value p to a patient-rotation angle $\alpha$.

In Step V, output-data O is generated based on the determined patient-rotation angle $\alpha$. This could be a message for a user or commands designed to control an automatic positioning mechanism. The generated output-data O is then outputted.

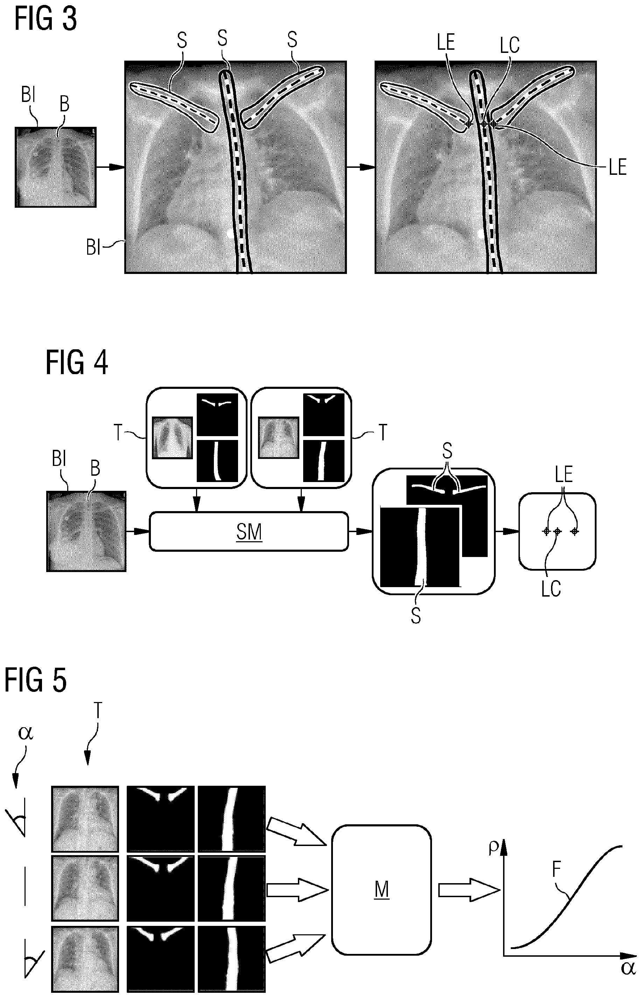

Figure 3 shows a segmentation procedure and an identification of landmarks. In general, this could be step 1 of figure 2. First, the base-image BI is provided showing a body-part B, in this example the chest. Then, the image information is segmented into three segments S, the left and right clavicle and the vertebral column. After that (right), landmarks LC, LE are identified, being a point on the vertebral column (center-landmark LC) and the left and right clavicle medial end (edge-landmarks LE).

Figure 4 shows the segmentation with a trained segmentation-model SM. Where figure 3 shows the base-image, figure 4 shows the process. First, the base-image BI is inputted in the segmentation-model SM that has been trained with training-data T comprising real or synthetic images and segmented bones. This segmentation-model SM segments the base-image BI. Then, the shape of the segments S is examined and landmarks LE, LC are added. This could be done by another trained machine learning model or by a conventional algorithm.

Figure 5 shows the training of a machine-learning-model M producing a mapping function F. training-data T with segmented images (preferably with landmarks LC, LE) and given patient-rotation angles $\alpha$ as ground truth are fed to the machine-learning model this model calculates the ratio-value p and creates pairs of ratio-value p and corresponding patient-rotation angles $\alpha$. These pairs are then collected in a graph and a polynomial function is fitted to these values (right) this function or its inverted function (depending on the fit) is then the mapping function F. In the shown example, the fitting function is the mapping function F.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The expression "a number of" means "at least one". The mention of a "unit" or a "device" does not preclude the use of more than one unit or device. Independent of the grammatical term usage, individuals with male, female or other gender identities are included within the term.

## Claims

1. A method for optimizing the positioning of a patient (P) for X-ray projection imaging, comprising the steps:

    - providing a base-image (BI) of a body part of the patient (P) positioned for acquiring the projection image,
    - identifying from a predefined set of landmarks a number of center-landmarks (LC) and a plurality of edge-landmarks (LE) in the base-image (BI), wherein each center-landmark (LC) lies between at least two edge-landmarks (LE) and wherein the equivalents of the landmarks in the patient (P) lie on a number of triangles inclined to the projection plane of the base-image (BI),
    - determining distances (D1, D2) of at least one center-landmark (LC) to the at least two edge-landmarks (LE) in the base-image (BI),
    - calculating a ratio-value (p) of the calculated distances (D1, D2),
    - determining a patient-rotation angle ($\alpha$) from the ratio-value (p) and a given mapping-function (F) that maps the ratio-value (p) to a patient-rotation angle ($\alpha$),
    - generating output-data (O) based on the determined patient-rotation angle ($\alpha$),
    - outputting the generated output-data (O).

2. The method according to claim 1, wherein parts of the body of the patient (P) are segmented (S) in the base-image (BI), especially organs and/or bones, preferably by using a machine-learning-model (M) trained for segmentation (S).

3. The method according to one of the preceding claims, wherein the distance (D1, D2) of the center-landmark (LC) to a first edge-landmark (LE) is A and the distance (D1, D2) of the center-landmark (LC) to a second edge-landmark (LE) is B and the ratio-value (p) R is calculated with the difference of A and B normed with the sum of A+B.

4. The method according to one of the preceding claims, wherein the patient-rotation angle ($\alpha$) is the angle of aberration of the patient (P) from a desired rotation-angle.

5. The method according to one of the preceding claims, wherein the patient-rotation angle ($\alpha$) is determined with a machine-learning-model (M) designed to output a patient-rotation angle ($\alpha$) based on an input of a ratio-value (p) that has been trained by inputting sets of ratio-values (p) with given real patient-rotation angles ($\alpha$) as ground truth values, preferably, wherein for training multiple synthetic X-ray images of at least one body-part (B) of a patient (P) are generated that are projected in various given rotation angles, wherein for each synthetic X-ray image landmarks are detected, a ratio-value (p) is calculated from distances (D1, D2) between the landmarks (LC, LE) and a patient-rotation angle ($\alpha$) is given as ground truth value based on the given rotation angle of the projection on the respective synthetic X-ray image.

6. The method according to one of the preceding claims, wherein for a plurality of certain ratio-values (p) matching patient-rotation angles ($\alpha$) are calculated and a matching function is fitted to the certain values,

    preferably wherein the matching function is a polynomial greater than second order, preferably greater than third order,
    particularly preferably wherein the inverse function of the matching function is calculated as mapping-function (F) for determining a patient-rotation angle ($\alpha$) from a ratio-value (p).

7. The method according to one of the preceding claims, wherein the body-part (B) of the patient (P) is the chest, the hip, the knee, shoulder, foot, the femur or the head.

8. The method according to one of the preceding claims, wherein the patient-rotation angle ($\alpha$) is calculated with a given mapping-function (F) providing values for a desired patient-rotation angle ($\alpha$) from a certain ratio-value ($\rho$),
    preferably wherein the mapping-function (F) is monotonically increasing or decreasing and crosses the origin.

9. The method according to one of the preceding claims, comprising the steps:

    - providing a base-image (BI) of a chest of the patient (P),
    - identifying at least a landmark on each clavicle and one landmark on the vertebral column in the base-image (BI),
    - calculating a distance (D1, D2) A of the landmark on the vertebral column to the landmark on the right clavicle and a distance (D1, D2) B of the landmark on the vertebral column to the landmark on the left clavicle in the base-image (BI),
- calculating a ratio-value (ρ) R of the calculated distances (D1, D2), preferably with the formula R = (A-B)/(A+B) or R = (B-A)/(A+B),
- determining a patient-rotation angle (α) from the ratio value, preferably by using a given mapping-function (F),
- generating an output based on the determined patient-rotation angle (α),
- outputting the generated output.

10. A device (6) for optimizing the positioning of a patient (P) for X-ray projection imaging, comprising:

   - a data-interface (7), designed for receiving a base-image (BI) of a body part of the patient (P) positioned for acquiring the projection image,
   - a landmark-unit (8), designed for identifying from a predefined set of landmarks a number of center-landmarks (LC) and a plurality of edge-landmarks (LE) in the base-image (BI), wherein each center-landmark (LC) lies between at least two edge-landmarks (LE) and wherein the equivalents of the landmarks in the patient (P) lie on a number of triangles inclined to the projection plane of the base-image (BI),
   - a distance-unit (D1, D2) (9), designed for determining distances (D1, D2) of a center-landmark (LC) to at least two edge-landmarks (LE) in the base-image (BI),
   - a ratio-unit (10), designed for calculating a ratio-value (ρ) of the calculated distances (D1, D2),
   - a correction-unit (11), designed for determining a patient-rotation angle (α) from the ratio value and a given mapping-function (F) that maps the ratio-value (ρ) to a patient-rotation angle (α),
   - an output-unit (12), designed for generating an output based on the determined patient-rotation angle (α),
   - a data-interface (7), designed for outputting the generated output.

11. The device (6) according to claim 10, comprising a function-unit (13) designed for

   generating a mapping-function (F) from multiple calculations of patient-rotation angles (α) from inputted ratio-values (ρ),
   preferably wherein the device (6), especially the function unit, comprises a machine-learning-model (M), trained for calculating a patient-rotation angle (α) from an inputted ratio-value (p).

12. A medical X-ray-system (1) comprising a device (6) according to claim 10 or 11 and/or designed for performing the method according to one of claims 1 to 9.

13. A computer program product comprising instructions which, when the program is executed by a computer, cause the computer to carry out the steps of the method of any of claims 1 to 9.

14. A computer-readable storage medium comprising instructions which, when executed by a computer, cause the computer to carry out the steps of the method of any of claims 1 to 9.

# FIG 1

# FIG 2

FIG 3

FIG 4

FIG 5

| | Europäisches Patentamt European Patent Office Office européen des brevets | **EUROPEAN SEARCH REPORT** | Application Number EP 24 19 8082 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/375120 A1 (VON BERG JENS [DE] ET AL) 24 November 2022 (2022-11-24) * paragraph [0002] - paragraph [0003] * * paragraph [0054] * * paragraph [0011] * * paragraph [0087] - paragraph [0088] * ----- | 1,4-14 | INV. A61B6/04 A61B6/00 A61B6/58 G06T7/73 |
| X | EP 3 644 273 A1 (KONINKLIJKE PHILIPS NV [NL]) 29 April 2020 (2020-04-29) * paragraph [0069] * ----- | 1-14 | |
| X | CN 115 359 879 A (BEIJING SMARTTREE MEDICAL TECH CO LTD) 18 November 2022 (2022-11-18) * the whole document * ----- | 1-14 | |
| | | | TECHNICAL FIELDS SEARCHED (IPC) A61B G06T |

The present search report has been drawn up for all claims

| Place of search | Date of completion of the search | Examiner |
|---|---|---|
| Munich | 11 February 2025 | Ordavo, Ivan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 8082

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2025

| Patent document cited in search report | | Publication date | Patent family member(s) | | Publication date |
|---|---|---|---|---|---|
| US 2022375120 | A1 | 24-11-2022 | CN 114650773 | A | 21-06-2022 |
| | | | EP 3815609 | A1 | 05-05-2021 |
| | | | EP 4051117 | A1 | 07-09-2022 |
| | | | US 2022375120 | A1 | 24-11-2022 |
| | | | WO 2021084041 | A1 | 06-05-2021 |
| EP 3644273 | A1 | 29-04-2020 | EP 3644273 | A1 | 29-04-2020 |
| | | | WO 2020083764 | A1 | 30-04-2020 |
| CN 115359879 | A | 18-11-2022 | NONE | | |

EPO FORM P0459

For more details about this annex : see Official Journal of the European Patent Office, No. 12/82